# EUROPÄISCHE PATENTSCHRIFT

(11) **EP 4 179 221 B1**
(45) Veröffentlichungstag und Bekanntmachung des Hinweises auf die Patenterteilung: **22.10.2025**
(21) Anmeldenummer: 21743430.7
(22) Anmeldetag: 09.07.2021
(51) Int. Cl.: F16B 23/00, B25B 15/00, F16B 25/00, F16B 25/10, F16B 35/06, A61B 17/70, A61B 17/86

(54) **WERKZEUGANSATZSTELLE MIT AUSRICHTHILFE**
TOOL ATTACHMENT POINT WITH ORIENTATION AID
POINT DE FIXATION D'OUTIL AVEC AIDE À L'ORIENTATION

(30) Priorität: 11.07.2020 DE 102020004179
(43) Veröffentlichungstag der Anmeldung: 17.05.2023
(73) Patentinhaber: Ortho Hub Ventures UG (Haftungsbeschränkt), 70794 Filderstadt (DE)
(72) Erfinder: HEUER, Frank, 70794 Filderstadt (DE)
(74) Vertreter: Stolmár & Partner Patentanwälte PartG mbB
(86) Internationale Anmeldenummer: PCT/EP2021/069204
(87) Internationale Veröffentlichungsnummer: WO 2022/013108

(56) Entgegenhaltungen:
- US-A- 6 016 727
- US-A1- 2019 380 747
- US-B2- 6 904 833

## Beschreibung

### Stand der Technik

Zum effizienten Arbeiten im Handwerk zählt vor allem das Einsparen von Zeit. Einer der häufigsten Schritte im Handwerk ist das Setzen von Schraubenelementen jeglicher Art. Eine zeitliche Einsparung kann insbesondere beim Zusammenfügen einer Schraube mit einem Schraubendreher erreicht werden. Ein wesentlicher Schritt beim Ansetzen eines Schraubendrehers mit einer Schraube ist es, dass der Werkzeugantrieb des Schraubendrehers genau zur rotatorischen Ausrichtung der Werkzeugansatzstelle der Schraube passen muss. Dies gelingt nicht immer auf Anhieb und ist für den Anwender nicht intuitiv. Dadurch kann dieser Schritt, auch durch die hohe Anzahl von Wiederholungen, enorm zeitraubend sein. Sollte unter schweren Sichtbedingungen bzw. gearbeitet werden, kann sich das Andocken des Schraubendrehers an die Schraube teilweise auch fehlerbehaftet sein und zur Zerstörung der Werkzeugansatzstelle führen.

Aus US3584667A sind Vielzahnrund-Profile für das Antrieben von Schraubelementen bekannt, die auch als Torx^{®} bezeichnet werden. Mit Hilfe eines Vielzahnrunds können höhere Drehmomente auf den Schraubenschaft übertragen werden und sind deshalb bevorzugt im Handwerk anzutreffen. Allerdings ist dem Stand der Technik keine Ausrichthilfe zu entnehmen, die eine rotatorische Ausrichtung eines Schraubendrehers beim Einführen des Schraubendrehers in ein Schraubenelement mit Torx^{®} ähnlicher Werkzeugansatzstelle unterstützt.

Dies soll mit der hier vorgestellten Erfindung gelöst werden. Aus den Grundlagen der Mechanik ist bekannt, dass das Drehmoment zum Einschrauben einer Schraube immer höher ist, als das Drehmoment zum Ausschrauben einer Schraube. Deshalb ist es notwendig, die Kontaktflächen einer Werkzeugansatzstelle zu einem Schraubendreher in Eindreh-Richtung zu maximieren, wobei gleichzeitig die gegenüberliegenden Kontaktflächen in Ausdreh-Richtung kleiner vorgesehen sein können. Die verkleinerten Kontaktflächen in Ausdreh-Richtung ermöglichen Bauraum für eine entsprechende Ausrichthilfe für einen Schraubendreher.

Die US 6,904,833 B2 offenbart ein asymmetrisches Schraubenschlüssel- und Befestigungssystem, das radial verlaufende Keilverzahnungen mit gegenüberliegenden Oberflächen umfasst, deren innere Enden in Bezug auf einen Radius, der die jeweiligen inneren Enden schneidet, um unterschiedliche Beträge geneigt sind, um das Drehmoment zwischen der Anzugs- und Löserichtung zu ändern. Diese Konfiguration macht es entweder einfacher oder schwieriger, das Befestigungselement zu lösen als es festzuziehen, je nachdem, wie stark die gegenüberliegenden Oberflächen der Keilverzahnungen geneigt sind.

Die US 2019/0380747 A1 offenbart ein Implantationsset zum Implantieren einer Schraube in einen Menschen bzw. Tierkörper umfasst ein Werkzeug und eine Schraube zur Aufnahme des Werkzeugs. Die Schraube weist einen Werkzeugaufnahmebereich auf in den ein Drehmomentübertragungsbereich des Werkzeugs zur Drehmomentübertragung eingesetzt wird. An der Werkzeugaufnahme ist mindestens ein geneigter Einführbereich ausgebildet, um das Werkzeug beim Einbringen der Schraube zu führen. Das Werkzeug hat mindestens eine darauf abgestimmte Zentrierfläche die auf den geneigten Einführbereich abgestimmt ist, so dass beim Eingreifen der Zentrierfläche mit der schrägen Einführung ein Form- und Kraftschluss entsteht.

Die US 6,016,727 offenbart ein Befestigungselement mit einem versenkten Antriebseinsatz und ein Antriebswerkzeug mit einem zusammenwirkenden Antriebskopf. Der Sockel hat eine zylindrische Innenwand, die durch eine Vielzahl von Antriebsecken unterbrochen ist. Der Antrieb hat einen konischen Antriebskopf mit mehreren geschärften Antriebskanten, die für den Eingriff in die Antriebsecken geeignet sind. Das konische Antriebsende kann axial in die Antriebsbuchse gedrückt werden, so dass die mehreren Antriebskanten reibschlüssig mit den Antriebsecken in Eingriff kommen, um das Befestigungselement am Antrieb zu halten und die Drehmomentübertragung zu verbessern. Die zylindrischen Wände des Sockels und die Wände des Schraubendrehereinsatzes zwischen den Antriebskanten wirken zusammen, um während der Positionierung innerhalb des Sockels einen Flüssigkeitskanal für den Flüssigkeitsdurchgang zu definieren. Die US 2020139523 A1. offenbart ein Schraubelement, das eine zentrale Öffnung mit einer Eindreh-Wandung und einer Ausdreh-Wandung besitzt, wobei die Fläche der Eindreh-Wandung größer als die Fläche der Ausdreh-Wandung ist.

### Darstellung der Erfindung

Die Aufgabe wird erfindungsgemäß gelöst durch ein Schraubenelement nach Anspruch 1. Weitere, die Erfindung ausgestaltende Merkmale sind in den Unteransprüchen enthalten.

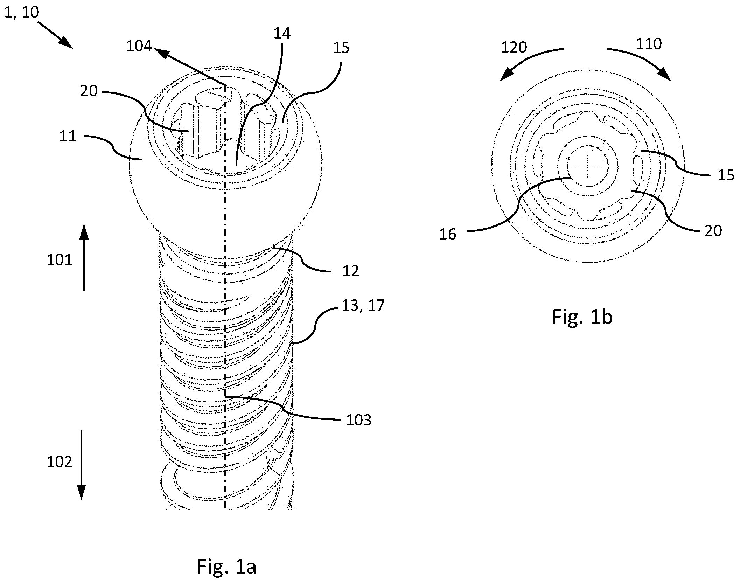

Für das erfindungsgemäße Schraubenelement (10), sind raumzuweisende Koordinatenreferenzen definiert, wie zum Beispiel die proximale Richtung (101) (zum Anwender hinzeigend), die distale Richtung (102) (vom Anwender wegzeigend), die sich entlang einer Zentralachse (103) ausdehnen. Von der Zentralachse (103) nach außen abgehend definiert sich die radiale Ausbreitung (104). Die umfängliche Ausbreitung ist durch einen konstanten Radius und entlang eines variablen Umfangswinkels definiert (Fig. 1a).

Verwendet wird das erfindungsgemäße Schraubenelement (10) für die Fixierung von jeglichen Dingen und Materialien. Es besteht dabei aus einem Schaft (13) mit einem Außengewinde (17) und eine von radial innen ausgehend Werkzeugansatzstelle (20). Das Außengewinde gibt dabei einen Drehsinn für das Einschrauben und Ausschrauben des Schraubenelements (10) vor. In Abhängigkeit dieses Drehsinns sind zwei weitere Raumrichtungen definiert; die Einschraub-Richtung (110) und die Ausschraub-Richtung (120) (Fig. 1b).

Das Schraubenelement kann eine längliche Schraube mit einem Kopf sein, der einen Antriebsabschnitt umfasst, welcher hier als Werkzeugansatzstelle (20) definiert ist. Es kann sich beispielsweise auch um eine Madenschraube handeln, die als Verriegelungselement verwendet wird. Im Allgemeinen kann das Schraubenelement in Fällen mit schlechter oder fehlender Sicht auf die Einsatzstelle verwendet werden, in denen die Position einer bereits platzierten Schraube angepasst werden muss. In einer bevorzugten Ausführungsform besitzt das Schraubenelement (10) einen Kopf (11), einen Halsbereich (12) und einen Schaft-Bereich (13) mit Außengewinde (18) und die Werkzeugansatzstelle (20) wird im Kopf (11) bereitgestellt (Fig. 1a). Die Werkzeugansatzstelle (20) ist in proximaler Richtung (101) offen und mündet optional in einer konzentrischen kegelartigen Ausnehmung (15). Der Kopf (11) ist vorzugsweise als Linsen-, Zylinder-, Schräg- oder als Kugelkopf ausgebildet. Es sind aber auch eine Zusammensetzung aus unterschiedlichen Rundungen und Flächen denkbar. Hauptmerkmal des Kopfes ist es, dass der Kopf (11) einen größeren Außendurchmesser als der Halsbereich (12) aufweist. Vorzugsweise ist die Werkzeugansatzstelle (20) als Sacklock ausgebildet und nach distaler Richtung (102) von einer Wandung (14) begrenzt ist. Optional kann diese Wandung (14) als Schräge ausgebildet sein, die kegelartig in zunehmender distaler Richtung (102) nach radial innen verläuft.

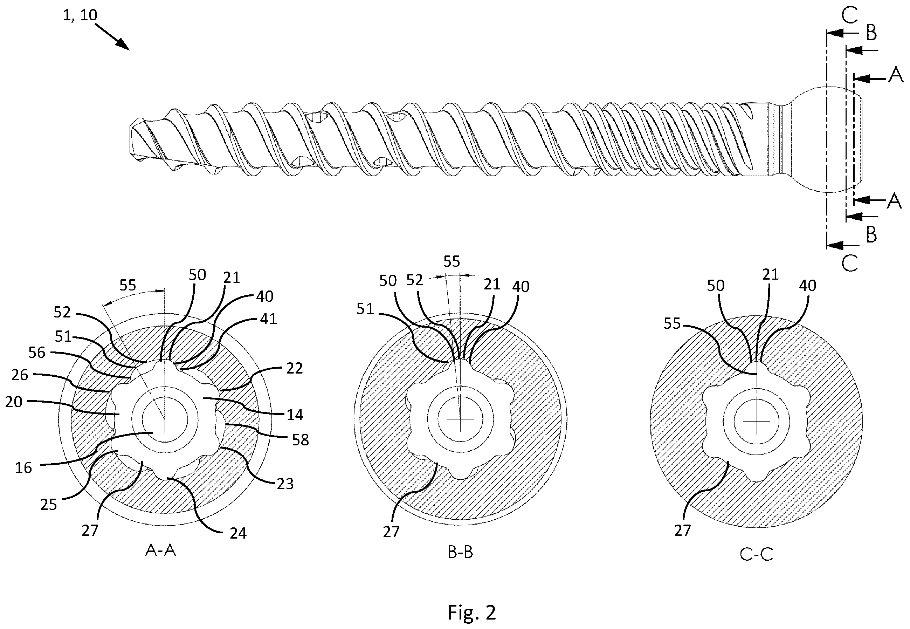

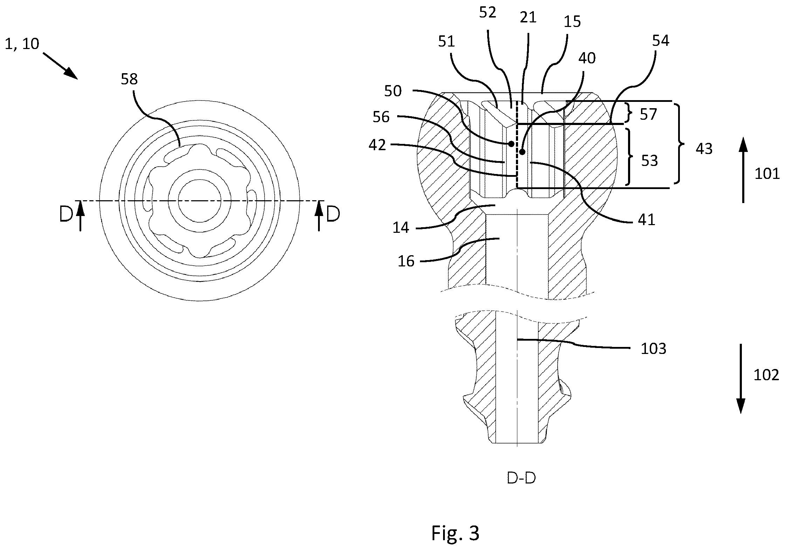

Ergänzend ist zu erwähnen, dass das Schraubenelement (10) eine von radial innen ausgehende Werkzeugansatzstelle (20) besitzt und darin eine zentrale Öffnung (27) vorgesehen ist. In der Wand dieser Öffnung (27) sind mindestens fünf nach radial außen (104) und hauptsächlich parallel zur Schraubenachse (103) gerichtete Zahnprofile (z.B. 21, 22, 23, 24, 25, 26) ausgebildet (Fig. 2). Vorzugsweise sind die Zahnprofile als konkave Wandungen gestaltet, so dass sich ein Torx^{®}-ähnliches oder genormtes Torx^{®}-Profil ergibt. Optimalerweise befinden sich Übergangsradien (56, 41) zwischen den Zahnprofilen (z.B. 21) und der zentralen Öffnung (27). Für jedes Zahnprofil kann die Zahnprofilfläche (z.B. 21) eine Eindreh-Wandung (40) in Eindreh-Richtung (110) und eine Ausdreh-Wandung (50) in Ausdreh-Richtung (120) unterteilen werden. Zur Unterteilung dieser Wandungen (40 und 50) dient eine gedachte Trennlinie (42) in Fig. 3. Ein wesentliches Merkmal der Erfindung ist, dass die Fläche der Eindreh-Wandung (40) größer ist als die Fläche der Ausdreh-Wandung (50). Somit hat ein Schraubendreher (60) mit einem komplementären Zahnprofil die volle Eingriff-Höhe in Eindreh-Richtung. In die weniger belastete Ausdreh-Richtung ist die Kontaktfläche zwischen Schraubendreher und Schraubenelement kleiner.

In Fig. 3 sind die erfindungsgemäßen Merkmale dargestellt. Zu erkennen ist, dass die Werkzeugansatzstelle (20) entlang der longitudinalen Achse (103) in mindestens zwei Abschnitte (53, 57) einteilbar ist, wobei von distaler Richtung kommend ein erster Abschnitt (53) ausgebildet ist und bis zur Höhe einer Abschnittstrennebene (54) die Flächen der Eindreh- und Ausdreh-Wandungen (40, 50) von jedem Zahnprofil (z.B. 21...26) in etwa gleich groß sind. Des Weiteren verläuft die Eindreh-Wandung (40) auf gesamter Höhe beider Abschnitte (43, 53 und 57) hauptsächlich parallel zur Schraubenachse (103). Im zweiten Abschnitt (57) ist eine Führungs-Wandung (51) ausgehend von der Abschnittstrennebene (54) ausgebildet, wobei die Führungs-Wandung (51) an die Ausdreh-Wandung (50) des ersten Abschnitts (53) ansetzt und diese Führungs-Wandung (51) in proximaler Richtung (101) von der Eindreh-Wandung (40) zunehmend beabstandet ist. Die zunehmende Beabstandung zwischen der Führungs-Wandung (51) und Eindreh-Wandung (40) verläuft hauptsächlich entlang des Umfangs in Ausdreh-Richtung (120) und dadurch ergibt sich eine in Umfangsrichtung erstreckende Wandung (52).

Zusammengefasst heißt dies, dass im ersten Abschnitt (53) eine herkömmliche Werkzeugansatzstelle vorgesehen ist, die symmetrische und hauptsächlich parallel zur Schraubenachse (103) ausgerichtete Zahnprofile besitzt (z.B. 21...26). Im zweiten Abschnitt (57) sind die Wandungen so angeordnet, dass sie die Ausrichthilfe für den Schraubendreher (60) bereitstellen. Die Ausrichtunghilfe ist in Fig. 2 im Schnitt quer zur Schraubachse (103) auf Höhe des zweiten Abschnitts (57) dargestellt. Dabei ist zu erkennen, dass die Wandungen (51, 52 und 40) eine Schnitt-Kontur erzeugen, die näherungsweise einem rotatorischen Langloch (58) entspricht und durch einen Öffnungswinkel (55) definierbar ist. Der Öffnungswinkel (55) besitzt einen maximalen Winkel zwischen 10° bis 60°, vorzugsweise jedoch 20° bis 50°. Dieser Winkel (55) verkleinert sich von proximaler Richtung (101) nach distaler Richtung (102), wobei dieser Winkel (55) im ersten Abschnitt (53) konstant bleibt. Diese Merkmale sind anhand der unterschiedlichen Schnittebenen in Fig. 2 illustriert.

Vorzugsweise besitzen die Abschnitte (53 und 57) eine unterschiedliche Höhe. Denkbar sind beispielsweise; dass die Höhe des ersten Abschnitts (53) größer ist als die Höhe für den zweiten Abschnitt (57), oder die Höhe des zweiten Abschnitts (57) größer ist als die Höhe für den ersten Abschnitt (53), oder auch dass die Höhen der beiden Abschnitte (53) und (57) in etwa gleich sind.

Die in Fig. 3 dargestellte Kanülierung (103) besitzt einen optionalen Charakter und ist für die erfindungsgemäßen Ausführungsformen nicht zwangsweise notwendig.

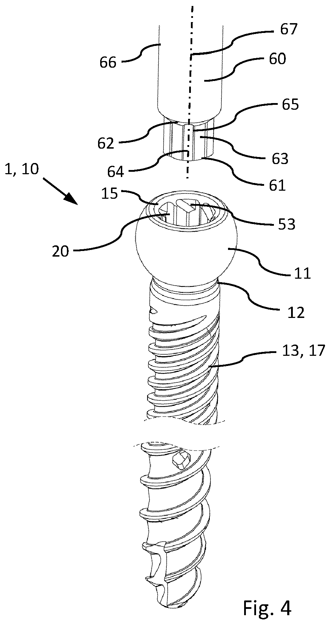

In Fig. 4 dargestellt ist ein typischer Schraubendreher (60) mit einem Schaft (66), welcher eine longitudinale Schraubendreherachse (67) definiert. Am distalen Ende (61) befindet sich eine Antriebseinheit (65) mit Zähnen (64) und ein Kern (63). Die Antriebseinheit (65) mündet in proximaler Richtung in einem kegelartigen Auslauf (62). Die bereits erwähnten Abschnitte (53, 57) der Werkzeugansatzstelle (20) des Schraubenelements (10) können optional in eine kegelartige konzentrische Ausnehmung (15) in proximaler Richtung (101) münden (Fig. 4). Die kegelartige Ausnehmung (15) hat zwei Funktionen. Einerseits werden bei Erstkontakt des Schraubendrehers (60) mit dem Schraubenelement (10) die Schraubenachse (103) mit der Achse (67) des Schraubendrehers (60) orthograd zueinander ausgerichtet indem das distale Ende (61) entlang des Konus (15) zur Zentralachse (103) des Schraubenelements (10) geführt wird. Anderseits wird bei voll eingeführtem Schraubendreher (60) ein flächiger Kontakt zwischen der kegelartigen Ausnehmung (14) und dem kegelartigen Auslauf (62) hergestellt, der für eine belastbare orthograde Ausrichtung des Schraubenelements (10) im Bezug zum Schraubendreher (60) sorgt.

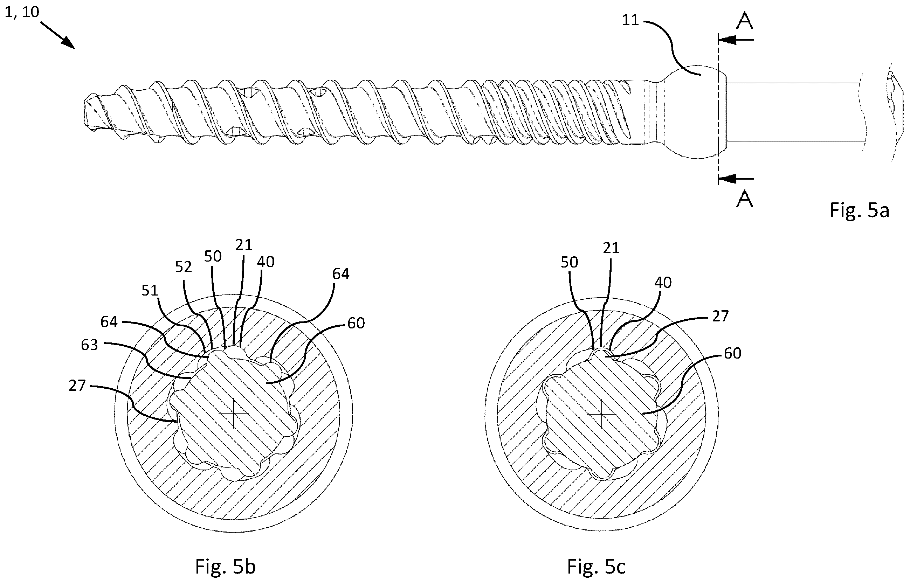

Ein weiteres Merkmal ist, dass der Schraubendreher (60) eine dem ersten Abschnitt (53) komplementär ausgebildete Antriebseinheit (65) mit Zähnen (64) besitzt (Fig. 5c), welche sich hauptsächlich parallel der Schraubendreher-Zentralachse (67) erstreckt und das Schraubenelement (10) im Bereich des zweiten Abschnitts (57) Führungswandungen (51) besitzt, welche die Zähne (64) des Schraubendrehers (60) rotatorisch um die Schraubendreher-Zentralachse (67) ausrichten (Fig. 5b), so dass für die Zähne (64) des Schraubendrehers (60) eine größere Kontaktfläche in Eindreh-Richtung (110) als in Ausdreh-Richtung (120) bereitgestellt wird.

### Kurze Beschreibung der Zeichnungen zeigen

Fig. 1a eine Schrägansicht des erfindungsgemäßen Schraubenelements.
Fig. 1b die Draufsicht auf die Werkzeugansatzstelle.
Fig. 2 zeigt eine Seitenansicht des erfindungsgemäßen Schraubenelements und drei an unterschiedlicher Position vorgesehene Schnitte durch die Werkzeugansatzstelle.
Fig. 3 illustriert einen Schnitt in der Seitenansicht der Werkzeugansatzstelle.
Fig. 4 stellt das Zusammenspiel aus Schraubenelement und Schraubendreher dar.
Fig. 5a zeigt eine Seitenansicht gemäß Fig. 4.
Fig. 5b zeigt im Schnitt, wie der Schraubendreher beim Einsetzen in die Werkzeugansatzstelle in einer beliebigen rotatorischen Position geführt und ausgerichtet wird, und
Fig. 5c zeigt den Zustand beim Einsetzen des Schraubendrehers, sobald die Antriebseinheit des Schraubendrehers den Abschnitt zur Ausrichtung passiert hat und im Eingriff mit dem Vielzahnrund ist.

## Patentansprüche

1. Schraubenelement (10) zum effizienten Arbeiten im Handwerk bestehend aus einem Schaft (13) mit einem Außengewinde (17) und eine sich am Schaft (13) entlang erstreckende longitudinalen Achse (103) und dadurch eine distale (102) und eine proximale (101) Richtung definiert, sowie sich eine Eindreh-Richtung (110) und eine davon entgegengesetzte Ausdreh-Richtung (120) ergibt, und von radial innen ausgehend eine Werkzeugansatzstelle (20) besitzt und die Werkzeugansatzstelle (20) eine zentrale Öffnung (27) besitzt und in der Wand dieser Öffnung (27) mindestens fünf nach radial außen (104) und hauptsächlich parallel zur Schraubenachse (103) gerichtete Zahnprofile (z.B. 21, 22, 23, 24, 25, 26) ausgebildet sind, und sich für jedes Zahnprofil eine Eindreh-Wandung (40) in Eindreh-Richtung (110) und eine Ausdreh-Wandung (50) in Ausdreh-Richtung (120) unterteilen lässt, wobei die Fläche der Eindreh-Wandung (40) größer als die Fläche der Ausdreh-Wandung (50) ist, wobei die Werkzeugansatzstelle (20) entlang der longitudinalen Achse (103) in mindestens zwei Abschnitte einteilbar ist, wobei von distaler Richtung kommend ein erster Abschnitt (53) ausgebildet ist und bis zur Höhe einer Abschnittstrennebene (54) die Flächen der Eindreh- und Ausdreh-Wandungen (40, 50) von jedem Zahnprofil (z.B. 21...26) in etwa gleich groß sind, wobei die Werkzeugansatzstelle (20) entlang longitudinaler Richtung (103) in mindestens zwei Abschnitte einteilbar (53, 57) ist und die Eindreh-Wandung (40) auf gesamter Höhe beider Abschnitte (43, 53 und 57) hauptsächlich parallel zur Schraubenachse (103) verläuft und wobei 11 im zweiten Abschnitt (57) eine Führungs-Wandung (51) ausgehend von der Abschnittstrennebene (54) ausgebildet ist, wobei die Führungs-Wandung (51) an die Ausdreh-Wandung (50) des ersten Abschnitts (53) ansetzt und diese Führungs-Wandung (51) in proximaler Richtung (101) von der Eindreh-Wandung (40) zunehmend beabstandet ist.

2. Schraubenelement (10) nach Anspruch 1
**dadurch gekennzeichnet,**
**dass** die zunehmende Beabstandung zwischen der Führungs-Wandung (51) und Eindreh-Wandung (40) hauptsächlich entlang des Umfangs in Ausdreh-Richtung (120) verläuft und sich dadurch eine in Umfangsrichtung erstreckende Wandung (52) ergibt.

3. Schraubenelement (10) nach einem der vorhergehenden Ansprüche, **dadurch gekennzeichnet, dass** im Schnitt quer zur Schraubachse (103) auf Höhe des zweiten Abschnitts (57) die Wandungen (51, 52 und 40) eine Schnitt-Kontur besitzen, die näherungsweise einem rotatorischen Langloch (58) entspricht und durch einen Öffnungswinkel (55) definierbar ist.

4. Schraubenelement (10) nach einem der vorhergehenden Ansprüche, **dadurch gekennzeichnet, dass** der Öffnungswinkel (55) einen maximalen Winkel zwischen 10° bis 60°, vorzugsweise jedoch 20° bis 50° besitzt, und sich dieser Winkel (55) nach distaler Richtung (102) verkleinert.

5. Schraubenelement (10) nach einem der vorhergehenden Ansprüche, **dadurch gekennzeichnet, dass** die Höhe des ersten Abschnitts (53) größer ist als die Höhe für den zweiten Abschnitt (57).

6. Schraubenelement (10) nach einem der vorhergehenden Ansprüche, **dadurch gekennzeichnet, dass** die Höhe des zweiten Abschnitts (57) größer ist als die Höhe für den ersten Abschnitt (53).

7. Schraubenelement (10) nach einem der vorhergehenden Ansprüche, **dadurch gekennzeichnet, dass** die Höhen der beiden Abschnitte (53) und (57) in etwa gleich sind.

8. Schraubenelement (10) nach einem der vorhergehenden Ansprüche, **dadurch gekennzeichnet, dass** die Werkzeugansatzstelle (20) in proximaler Richtung (101) offen ist und in einer konzentrischen kegelartigen Ausnehmung (15) mündet.

9. Schraubenelement (10) nach einem der vorhergehenden Ansprüche, **dadurch gekennzeichnet, dass** die Werkzeugansatzstelle (20) nach distaler Richtung (102) von einer Wandung (14) begrenzt ist.

10. Schraubenelement (10) nach Anspruch 9,
**dadurch gekennzeichnet,**
**dass** die Wandung (14) als Schräge in zunehmender distaler Richtung (102) nach radial innen verläuft.

11. Schraubenelement (10) nach einem der vorhergehenden Ansprüche, **dadurch gekennzeichnet, dass** die zentrale Öffnung (27) einen konzentrisch zylindrischen Verlauf besitzt.

12. Schraubenelement (10) nach einem der vorhergehenden Ansprüche, **dadurch gekennzeichnet, dass** das Schraubenelement (10) zusätzlich einen Kopf (11), einen Halsbereich (12) und einen Schaft-Bereich (13) mit einem Außengewinde (18) aufweist und die Werkzeugansatzstelle (20) im Kopf (11) bereitgestellt wird.

13. System bestehend aus einem Schraubendreher (60) und mindestens einem Schraubenelement (10), nach einem der vorhergehenden Ansprüche, **dadurch gekennzeichnet, dass** das Schraubendreher (60) eine dem ersten Abschnitt (53) komplementär ausgebildete Antriebseinheit (65) mit Zähnen (64) besitzt, welche sich hauptsächlich parallel der Schraubendreher-Zentralachse (67) erstreckt und das Schraubenelement (10) im Bereich des zweiten Abschnitts (57) Führungswandungen (51) besitzt, welche die Zähne (64) des Schraubendrehers (60) rotatorisch um die Schraubendreher-Zentralachse (67) ausrichten, so dass für die Zähne (64) des Schraubendrehers (60) eine größere Kontaktfläche in Eindreh-Richtung (110) als in Ausdreh-Richtung (120) bereitgestellt wird.

## Claims

1. Screw element (10) for working efficiently in handicraft comprising a shaft (13) with an outside screw thread (17) and a longitudinal axis (103) extending along the shaft (13) and thereby defining a distal direction (102) and a proximal direction (101), and an insertion direction (110) and an opposite removal direction (120), and has a tool attachment point (20) starting radially inwards and the tool attachment point (20) has a central opening (27) and in the wall of this opening (27) at least five tooth profiles (e.g. 21, 22, 23, 23) directed radially outwards (104) and mainly parallel to the screw axis (103) and for each tooth profile an insertion wall (40) in insertion direction (110) and a removal wall (50) in removal direction (120) can be classified, wherein the surface of the insertion wall (40) is greater than the surface of the removal wall (50), wherein the tool attachment point (20) is separable along the longitudinal axis (103) into at least two sections, wherein a first section (53) is formed coming from the distal direction and the surfaces of the insertion and removal walls (40, 50) of each tooth profile (e.g. 21...26) are approximately equal in size up to the height of a section parting plane (54), wherein the tool attachment point (20) is separable along the longitudinal direction (103) into at least two sections (53, 57) and the insertion wall (40) extends mainly parallel to the screw axis (103) over the entire height of both sections (43, 53 and 57) and wherein a guiding wall (51) is formed in the second section (57) starting from the section parting plane (54), wherein the guiding wall (51) adjoins the removal wall (50) of the first section (53) and said guiding wall (51) is increasingly spaced apart in proximal direction (101) from the insertion wall (40).

2. Screw element (10) according to claim 1, **characterized in that** the increasing spacing between the guiding wall (51) and insertion wall (40) is mainly along the circumference in removal direction (120), resulting in a wall (52) extending in the peripheral direction.

3. Screw element (10) according to any of the preceding claims, **characterized in that**, in cross-section transverse to the screw axis (103) at the level of the second section (57), the walls (51, 52 and 40) have a sectional contour which corresponds approximately to a rotational slotted hole (58) and can be defined by an opening angle (55).

4. Screw element (10) according to any of the preceding claims, **characterized in that** the opening angle (55) has a maximum angle of between 10° and 60°, but preferably 20° to 50°, and said angle (55) decreases towards the distal direction (102).

5. Screw element (10) according to any of the preceding claims, **characterized in that** the height of the first section (53) is greater than the height for the second section (57).

6. Screw element (10) according to any of the preceding claims, **characterized in that** the height of the second section (57) is greater than the height of the first section (53).

7. Screw element (10) according to any of the preceding claims, **characterized in that** the heights of the two sections (53) and (57) are approximately equal.

8. Screw element (10) according to any of the preceding claims, **characterized in that** the tool attachment point (20) is open in the proximal direction (101) and ends in a concentric cone like recess (15).

9. Screw element (10) according to any of the preceding claims, **characterized in that** the tool attachment point (20) is bounded in distal direction (102) by a wall (14).

10. Screw element (10) according to claim 9, **characterized in that** the wall (14) extends as a slope in increasing distal direction (102) radially inwards.

11. Screw element (10) according to any of the preceding claims, **characterized in that** the central opening (27) has a concentric cylindrical course.

12. Screw element (10) according to any of the preceding claims, **characterized in that** the screw element (10) additionally comprises a head (11), a neck area (12) and a shaft area (13) with bone thread (18) and the tool attachment point (20) is provided in the head (11).

13. System consisting of a screw driver (60) and at least one screw element (10), according to any of the preceding claims, **characterized in that** the screw driver (60) has a drive unit (65) with teeth (64) complementary to the first section (53), said drive unit (65) extending mainly parallel to the central axis of the screw driver (67) and the screw element (10) has guiding walls (51) in the area of the second section (57), which align the teeth (64) of the screw driver (60) rotationally round the central axis of the screw driver (67), so that a larger contact surface is provided for the teeth (64) of the screw driver (60) in the insertion direction (110) than in the removal direction (120).

## Revendications

1. Élément vis (10) pour un travail efficace dans le métier composé d'une tige (13) avec un filetage extérieur (17) et un axe longitudinal (103) s'étendant le long de la tige (13) et définissant ainsi une direction distale (102) et une direction proximale (101), ainsi qu'une direction de vissage (110) et une direction de dévissage (120) opposée à celle-ci, et possédant un point de fixation d'outil (20) en partant radialement de l'intérieur et le point de fixation d'outil (20) possédant une ouverture centrale (27) et dans la paroi de cette ouverture (27) sont formées au moins cinq profilés dentés (par exemple 21, 22, 23, 24, 25, 26) orientés radialement vers l'extérieur (104) et principalement parallèlement à l'axe de vis (103) et pour chaque profilé denté on peut subdiviser une paroi de vissage (40) dans la direction de vissage (110) et une paroi de dévissage (50) dans la direction de dévissage (120), la surface de la paroi de vissage (40) étant plus grande que la surface de la paroi de dévissage (50), le point de fixation d'outil (20) pouvant être divisé en au moins deux sections le long de l'axe longitudinal (103), une première section (53) étant formée en venant de la direction distale et jusqu'à la hauteur d'un plan de séparation de section (54) les surfaces des parois de vissage et de dévissage (40, 50) de chaque profilé denté (par exemple 21...26) sont à peu près de la même taille, le point de fixation d'outil (20) pouvant être divisé (53, 57) en au moins deux sections le long de la direction longitudinale (103) et la paroi de vissage (40) sur toute la hauteur des deux sections (43, 53 et 57) étant principalement parallèle à l'axe de vis (103) et une paroi de guidage (51) étant formée dans la deuxième section (57) à partir du plan de séparation de section (54), la paroi de guidage (51) étant fixée à la paroi de dévissage (50) de la première section (53) et cette paroi de guidage (51) étant de plus en plus espacée de la paroi de vissage (40) dans la direction proximale (101).

2. Élément vis (10) selon la revendication 1, **caractérisé en ce que** l'espacement croissant entre la paroi de guidage (51) et la paroi de vissage (40) s'étend principalement le long de la circonférence dans la direction de dévissage (120) et qu'il en résulte une paroi (52) s'étendant dans la direction circonférentielle.

3. Élément vis (10) selon l'une quelconque des revendications précédentes, **caractérisé en ce que**, en coupe transversale à l'axe de vis (103) au niveau de la deuxième section (57), les parois (51, 52 et 40) possèdent un contour de coupe qui correspond approximativement à un trou oblong de rotation (58) et qui peut être défini par un angle d'ouverture (55).

4. Élément vis (10) selon l'une quelconque des revendications précédentes, **caractérisé en ce que** l'angle d'ouverture (55) possède un angle maximal compris entre 10 ° et 60 °, mais de préférence entre 20 ° et 50 °, et cet angle (55) diminue dans la direction distale (102).

5. Élément vis (10) selon l'une quelconque des revendications précédentes, **caractérisé en ce que** la hauteur de la première section (53) est supérieure à la hauteur de la deuxième section (57).

6. Élément vis (10) selon l'une quelconque des revendications précédentes, **caractérisé en ce que** la hauteur de la deuxième section (57) est supérieure à la hauteur de la première section (53).

7. Élément vis (10) selon l'une quelconque des revendications précédentes, **caractérisé en ce que** les hauteurs des deux sections (53) et (57) sont sensiblement égales.

8. Élément vis (10) selon l'une quelconque des revendications précédentes, **caractérisé en ce que** le point de fixation d'outil (20) est ouvert dans la direction proximale (101) et débouche dans un évidement conique concentrique (15).

9. Élément vis (10) selon l'une quelconque des revendications précédentes, **caractérisé en ce que** le point de fixation d'outil (20) est délimité en direction distale (102) par une paroi (14).

10. Élément vis (10) selon la revendication 9, **caractérisé en ce que** la paroi (14) s'étend radialement vers l'intérieur sous forme de biseau dans la direction distale croissante (102).

11. Élément vis (10) selon l'une quelconque des revendications précédentes, **caractérisé en ce que** l'ouverture centrale (27) présente un développement cylindrique concentrique.

12. Élément vis (10) selon l'une quelconque des revendications précédentes, **caractérisé en ce que** l'élément vis (10) présente en outre une tête (11), une zone de col (12) et une zone de tige (13) avec un filetage extérieur (18) et le point de fixation d'outil (20) est prévu dans la tête (11).

13. Système composé d'un tournevis (60) et d'au moins un élément vis (10), selon l'une quelconque des revendications précédentes, **caractérisé en ce que** le tournevis (60) possède une unité d'entraînement (65) avec des dents (64), formée de manière complémentaire à la première section (53), qui s'étend principalement parallèlement à l'axe central (67) du tournevis et l'élément vis (10) possède, dans la zone de la deuxième section (57), des parois de guidage (51) qui alignent les dents (64) du tournevis (60) en rotation autour de l'axe central (67) du tournevis, de sorte qu'une plus grande surface de contact est prévue pour les dents (64) du tournevis (60) dans la direction de vissage (110) que dans la direction de dévissage (120).
